Europäisches Patentamt

European Patent Office

Office européen des brevets

(11) Veröffentlichungsnummer: **0 097 898**

**B1**

(12) EUROPÄISCHE PATENTSCHRIFT

(45) Veröffentlichungstag der Patentschrift: 20.01.88

(21) Anmeldenummer: 83105992.8

(22) Anmeldetag: 20.06.83

(51) Int. Cl.⁴: **C 08 J 9/42, C 12 N 11/08**

┌─────────────┐
│ E R R A T U M │
└─────────────┘

(SEITE, SPALTE, ZEILE)
(PAGE, COLUMN, LINE)
(PAGE, COLONNE, LIGNE)

| DIE TEXTSTELLE : TEXT PUBLISHED : LE PASSAGE SUIVANT : | Seite | Spalte | Zeile | LAUTET BERICHTIGT: SHOULD READ : DEVRAIT ETRE LU : |
|---|---|---|---|---|
| gemäß Anspruch 1 bis 4 | 2 | 2 | 16 | gemäß Anspruch 1 bis 5 |
| und kann z.N. | 3 | 3 | 05 | und kann z.B. |
| Unter Verwedung von | | 4 | 33 | Unter Verwendung von |
| ein Perpolymerisat | | | 38/39 | ein Perlpolymerisat |
| B) Sulfonierung des makroporö-sen Perlpolymerisats 20 g des.... | | | 51/52 | B) Sulfonierung des makroporösen Perlpolymerisats. 20 g des...... |
| C) Beladung des sulfonierten Perlpolymerisats mit Polyethy-lenimin 10 g des..... | | | 62/63 | C) Beladung des sulfonierten Perl-polymerisats mit Polyethylen-imin. 10 g des..... |
| D) Beladung des Trägers mit Enzym und Ermittlung der immobilisierten Enzymaktivität 0,2 g Träger..... | 4 | 5 | 08/10 | D) Beladung des Trägers mit Enzym und Ermittlung der immobilisierten Enzymaktivität. 0,2 g Träger..... |

Tag der Entscheidung über die Berichtigung )
Date of decision on rectification: ) 06.04.88 ........
Date de décision portant ) sur modification:

Ausgabe- und Ver-öffentlichungstag: )
Issue and publication ) 08.06.88 ...........
date:
Date d'edition et de ) publication:

Patbl.Nr.)

EPB no:) 88/23...

Bull. no:)

Europäisches Patentamt

European Patent Office

Office européen des brevets

(11) Veröffentlichungsnummer: **0 097 898**
**B1**

(12) EUROPÄISCHE PATENTSCHRIFT

(45) Veröffentlichungstag der Patentschrift: 20.01.88

(51) Int. Cl.⁴: **C 08 J 9/42, C 12 N 11/08**

(21) Anmeldenummer: 83105992.8

(22) Anmeldetag: 20.06.83

(54) Makroporöse, hydrophile Träger für Enzyme.

(30) Priorität: 26.06.82 DE 3223885

(43) Veröffentlichungstag der Anmeldung:
11.01.84 Patentblatt 84/02

(45) Bekanntmachung des Hinweises auf die
Patenterteilung:
20.01.88 Patentblatt 88/03

(84) Benannte Vertragsstaaten:
AT BE CH DE FR GB IT LI NL SE

(56) Entgegenhaltungen:
EP-A-0 049 385
FR-A-2 021 567
FR-A-2 307 817

CHEMICAL ABSTRACTS, Band 83, Nr. 14, 1975,
Seite 111, Nr. 116411c, Columbus, Ohio, US

(73) Patentinhaber: BASF Aktiengesellschaft
Carl-Bosch-Strasse 38
D-6700 Ludwigshafen (DE)

(72) Erfinder: Tschang, Chung-Ji, Dr.
Hinterbergstrasse 31
D-6702 Bad Duerkheim (DE)
Erfinder: Marcinowski, Stefan, Dr.
Kopernikusstrasse 49
D-6700 Ludwigshafen (DE)

Courier Press, Leamington Spa, England.

**Beschreibung**

Die Erfindung betrifft makroporöse, vernetzte und hydrophile Polymerisate und deren Verwendung zur Immobilisierung von Enzymen.

Immobilisierte, d.h. trägergebundende Enzyme, finden u.a. Anwendung in der medizinischen Analytik, bei der Herstellung pharmazeutischer Produkte, der Herstellung optisch aktiver Substanzen sowie bei der Herstellung von Nahrungsmitteln, beispielsweise der Isoglucose. Die Vorteile der Verwendung von immobilisierten Enzymen bestehen in ihrer Wiederverwendbarkeit, ihrer einfachen Abtrennung vom Substrat bzw. dessen Lösung, ihrer oftmals erhöhten Stabilität und damit Lebensdauer unter gegebenen Bedingungen im Vergleich zur löslichen Form, der Vermeidung einer Verunreinigung der Reaktionsprodukte sowie der Möglichkeit, kontinuierliche Reaktionen in Säulen oder ähnlichen Reaktoren durchzuführen.

Es ist eine Vielzahl von Möglichkeiten der Immobilisierung von Enzymen bekannt. Eine ausführliche Übersicht wird beispielsweise in Methods in Enzymology, Vol. XLIV, "Immobilized Enzymes", (Academic Press, 1976), und in Immobilizer Enzymes (I. Chibata; Kodansha Ltd./John Wiley & Sons, 1978) gegeben. Ein häufig beschriebener Weg ist die adsorptive, ionische oder kovalente Bindung von Enzymen an Träger. Dabei wird organisches und anorganisches Trägermaterial sowie solches nativen Ursprungs verwendet.

In den letzten Jahren wurden zahlreiche Träger beschrieben, die unter Verwendung von Polyethylenimin hergestellt wurden. Bei einigen wird als Gerüstmaterial Polyethylenimin verwendet, das mit funktionellen Gruppen zur kovalenten Bindung von Enzymen versehen wird (G. Manecke, S. Heydolph, Makromol. Chem. *182* (1981), 2641—2657, P. Grunwald et al., Naturwissenschaften *68* (1951), 525—526). In einem anderen Falle wird Polyethylenimin kovalent an Nylon gebunden und dient dann zur Bindung eines Maleinsäureanhydrid/Vinylmethylether-Copolymerisats, welches seinerseits die kovalente Bindung von Enzymen bewirkt (A. Sugitachi et. al., Thrombos. Haemostas. (Stuttgart), *39* (1978), 426—435). Eine dritte Gruppe von Polyethylenimin enthaltenden Trägern schließlich basiert auf anorganischen, teilweise mit Polymeren umhüllten Materialien, auf die Polyethylenimin aufgebracht und· dann mit einem Überschuß an Glutardialdehyd, Diisocyanaten oder anderen mehrfunktionellen Reagenzien vernetzt wird (J. Konecny, W. Voser, Biochim. Biophys. Acta *485* (1977), 367—378, DE—AS 26 05 797; US—PS 4 141 857; US—PS 4 268 419; US—PS 4 268 423). Allen diesen Trägern ist gemeinsam, daß das in bzw. auf ihnen vorhandene Polyethylenimin kovalent vernetzt ist. Hierzu ist ein zusätzlicher Arbeitsgang und, wie aus den obigen Angaben zu entnehmen ist, die Verwendung von toxischen Agenzien wie z.B. Diisocyanaten nötig. Ferner wird das Trägermaterial bei der Ausrüstung mit enzymbindenden Gruppen wie Isocyanaten oder Maleinsäureanhydrid-Gruppen feuchtigkeitsempfindlich und muß vor dem Einsatz unter Ausschluß von Wasser aufbewahrt werden.

Der vorliegenden Erfindung liegt die Aufgabe zugrunde, möglichst einfach herstellbare Träger zur dauerhaften Immobilisierung von Enzymen mit hoher enzymatischer Aktivität zu entwickeln, die toxische Substanzen möglichst vermeiden und im Einsatz problemlos handhabbar sind, d.h. ohne besondere Vorsichtsmaßnahmen gelagert werden können und gute mechanische und hydrodynamische Eigenschaften besitzen.

Die Lösung dieser Aufgabe besteht in den Trägern gemäß Anspruch 1 bis 4. Solche Träger werden durch Behandlung von makroporösen, vernetzten, sulfogruppenhaltigen Polymerisatteilchen mit Polyethylenimin erhalten. Dabei kann man überraschenderweise auf eine Vernetzung des Polyethylenimins verzichten, wodurch — was völlig unvorhersehbar war — die gebundene enzymatische Aktivität erhöht wird.

Die Polymerisate, die durch radikalische Polymerisation hergestellt werden, enthalten einpolymerisiert 10 bis 100, vorzugsweise 15 bis 40 Gew.% Divinylbenzol, 0 bis 90, vorzugsweise 60 bis 85 Gew.% Styrol, das am Kern auch ein- oder mehrfach $C_{1-4}$-alkyl-substituiert sein kann, sowie 0 bis 20, vorzugsweise 0 bis 10 Gew.% mit Divinylbenzol und Styrol copolymerisierbare Monomere wie beispielsweise Alkylester der Acryl- oder Methacrylsäure. Die Einarbeitung von wasserlöslichen Monomeren wie z.B. Acrylsäure, Methacrylsäure oder Vinylimidazol in kleinen Mengen (bis 10 Gew.%) ist ebenfalls möglich. Die besonders bevorzugten Polymerisate bestehen aus 30 bis 60 Gew.% technischem Divinylbenzol (enthält ca. 50 % Divinylbenzol, Rest überwiegend Ethylstyrol, daneben u.a. auch geringe Mengen Trivinylbenzol) und 70 bis 40 Gew.% Styrol.

Die Polymerisation des Monomeren(gemischs) wird in Form einer Suspensionspolymerisation mit Wasser als äußerer Phase durchgeführt, wobei ein Teilchendurchmesser von 0,05 bis 3 mm, bevorzugt 0,2 bis 0,8 mm, angestrebt wird. Zur Erzeugung der Makroporosität des Polymerisats wird ein Porenbildner verwendet. Als Porenbildner eignet sich eine die Polymerisation nicht störende, mit Wasser nicht mischbare, das Monomere lösende, aber das Polymere höchstens schwach quellende Flüssigkeit, beispielsweise Alkane mit 7 bis 12 C-Atomen, bevorzugt n-Octan oder Benzine mit einem Siedepunkt von mindestens 100°C. Die Menge an Porenbildner beträgt 50 bis 400 Gew.%, bezogen auf die Monomeren. Die obere Grenze der Porenbildner-Menge ergibt sich aus den mechanischen Eigenschaften des Polymerisats und ist vom jeweiligen Porenbildner abhängig. Wesentlich ist die Erzeugung eines Porenvolumens von 0,8 bis 4 $cm^3/g$, bevorzugt 1,2 bis 3,0 $cm^3/g$, quecksilberporosimetrisch gemessen, wobei die Durchmesser der Poren $2,5 \cdot 10^{-5}$ bis $2 \cdot 10^{-3}$ mm, bevorzugt $4 \cdot 10^{-5}$ bis $5 \cdot 10^{-4}$ mm betragen sollen. Der Porenbildner wird aus dem fertigen Polymerisat mit Hilfe eines niedrigsiedenden Lösungsmittels, z.B. Aceton, herausgewaschen, dann wird das Polymerisat getrocknet. Die Herstellung von makroporösen Perlpolymerisaten ist bekannt und kann z.N. der GB—PS 849 122 entnommen werden.

Selbstverständlich wäre es auch möglich, die Polymerisation im Block vorzunehmen. Dies brächte jedoch den Nachteil, daß das Polymerisat nachträglich zerkleinert werden müßte und dann infolge der unregelmäßigen Form der Teilchen schlechtere hydrodynamische Eigenschaften hätte.

Zur Erzielung einer höheren immobilisierten Enzymaktivität ist es vorteilhaft, die Oberfläche des Polymerisats einer mechanischen Nachbehandlung entsprechend EP—OS 49 385 zu unterziehen.

Um das Polymerisat hydrophil zu machen, wird es an der Oberfläche sulfoniert. Die Sulfonierung geschieht in an sich bekannter Weise unter Verwendung von Schwefelsäure oder Chlorsulfonsäure. Beschreibungen von Sulfonierungsmethoden findet man u.a. in D. Braun, H. Cherdron und W. Kern, "Praktikum der Makromolekularen Organischen Chemie", Dr. Alfred Hüthig Verlag, Heidelberg 1966, Seite 234; in Ullmanns Encyklopädie, 4. Aufl., Verlag Chemie, Weinheim — New York, Bd. 13, Seite 301 sowie in Houben-Weyl, "Methoden der Organischen Chemie", Georg Thieme-Verlag, Stuttgart 1963, Band XIV/2, S. 682 bis 685. Die Sulfonierung wird soweit geführt, daß die Polymerisatoberfläche ausreichend sauer und hydrophil wird für die anschließende Polyethyleniminbeladung.

Das Polyethylenimin wird auf das sulfonierte Polymerisat in wäßriger Lösung aufgebracht. Das Molekulargewicht des Polyethylenimins, das linear oder verzweigt sein kann, soll 1000 bis 50 000, bevorzugt 30 000 bis 45 000 betragen. Um eine mechanische Schädigung des Polymerisats durch osmotische Einwirkung zu vermeiden, sollte man das Polyethylenimin in mehreren Portionen zugeben, wobei die Lösungskonzentration am Anfang etwa 2, jedenfalls nicht über 3 Gew.% beträgt. Bei Verwendung der zehnfachen Menge an wäßriger Lösung, bezogen auf die Menge an sulfoniertem Polymerisat, beträgt die Polyethylenimin-Endkonzentration in der Lösung 1 bis 6 Gew.%. Das Optimum der Polyethyleniminbeladung kann in Vorversuchen durch Bestimmung der immobilisierbaren enzymatischen Aktivität leicht ermittelt werden. Das Aufbringen des Polyethylenimins erfolgt bei 0 bis 100°C, vorzugsweise bei Raumptemperatur in einem Zeitraum von 1 bis 12, bevorzugt 4 bis 8 Stunden. Wegen der geringen Konzentration des Polyethylenimins ist die Anwendung höherer Temperaturen zur Senkung der Viskosität der Lösung nicht nötig. Nach der Beladung mit 5 bis 30, vorzugsweise 10 bis 20 Gew.% Polyethylenimin kann der Träger mit Wasser gewaschen werden. Dies ist jedoch nicht zwingend erforderlich. Der Träger kann bis zur Enzymbeladung wahlweise in getrockneter oder nasser Form gelagert werden.

Das Beladen des Trägers mit Enzym geschieht mit einer wäßrigen Enzymlösung, die je nach Enzym zweckmäßig einen Puffer enthalten kann. Die Konzentration des Enzyms in der Lösung beträgt 1 bis 100 mg/ml, die Beladungsdauer 1 bis 80, vorzugsweise 10 bis 40 Stunden. Die Temperatur der Enzymlösung liegt — je nach Enzym — bei 0 bis 90°C. Nach der Beladung wird der Träger von überschüssigem Enzym durch Waschen befreit und als heterogener Katalysator mit enzymatischer Aktivität entweder im diskontinuierlichen oder kontinuierlichen Betrieb, z.B. in einer Säule, eingesetzt. Die Lagerfähigkeit des enzymbeladenen Trägers richtet sich nach dem Enzym. Im allgemeinen empfiehlt es sich, die Enzymbeladung kurz vor dem Einsatz vorzunehmen.

Es wird angenommen, daß das Enzym auf dem Träger ionisch gebunden wird, ohne daß damit andere Bindungsmechanismen — mit Ausnahme der kovalenten Bindung — prinzipiell ausgeschlossen werden sollen.

Der Träger zeichnet sich durch einfache Handhabung aus, da er weder durch Feuchtigkeit desaktiviert wird noch vor Gebrauch aktiviert werden muß. Er vermag eine hohe enzymatische Aktivität zu immobilisieren, besitzt ausgezeichnete hydrodynamische Eigenschaften und wird von Mikroorganismen nicht angegriffen.

## Beispiel 1

A) Herstellung des makroporösen Perlpolymerisats

Unter Verwedung von 50 g Styrol, 50 g technischem Divinylbenzol, 180 ml n-Octan, 1 g Lauroylperoxid als Radikalbildner, 500 ml Wasser sowie 1 g Polyvinylpyrrolidon als Suspensionshilfsmittel wurde in einem Kolben mit Rührer und Rückflußkühler unter Einleiten von Stickstoff ein Perpolymerisat hergestellt, wobei 4 Stunden auf 70°C und dann 4 Stunden auf 95°C erhitzt wurde. Das Polymerisat wurde nacheinander in Aceton, Wasser und Methanol gewaschen und dann bei 70°C im Vakuum getrocknet. Danach wurde das Polymerisat auf eine Teilchengröße von $3,1.10^{-2}$ cm bis $5.10^{-2}$ cm gesiebt. 25 g dieses gesiebten Polymerisats wurden sodann 3 Stunden lang in einer 250 ml Glasflasche maschinell heftig geschüttelt, danach erneut mit Aceton, Wasser und Methanol gewaschen und schließlich bei 70°C im Vakuum getrocknet.

B) Sulfonierung des makroporösen Perlpolymerisats 20 g des nach A) hergestellten Polymerisats wurden mit 300 ml konzentrierter Schwefelsäure 1 Stunde bei Raumtemperatur, dann 8 Stunden bei 100°C gerührt. Dann wurde das Polymerisat auf einer Fritte abgesaugt und zunächst in halbkonzentrierte Schwefelsäure und danach in Wasser übergeführt, unter mehrmaligem Wechseln des Wassers gründlich gewaschen und schließlich bei 70°C im Vakuum getrocknet. Ausbeute: 31,5 g.

C) Beladung des sulfonierten Perlpolymerisats mit Polyethylenimin 10 g des nach B) sulfonierten Polymerisats wurden in 90 ml wäßriger Lösung von 1,5 g Polyethylenimin mit einem Molekulargewicht von ca. 40 000 2 Stunden bei Raumtemperatur gerührt. Dann wurden nochmals 1,5 g Polyethylenimin, gelöst in 8,5 ml Wasser, zugesetzt und es wurde weitere 4 Stunden gerührt. Der Träger wurde auf einer Glasfritte abgesaugt, mit 100 ml Wasser gewaschen und dann bei 70°C im Vakuum getrocknet.

D) Beladung des Trägers mit Enzym und Ermittlung der immobilisierten Enzymaktivität 0,2 g Träger wurden in 20 ml Lösung von 100 mg Invertase (150 internat. Units/mg) in 0,005 M wäßriger Natriumacetatlösung (pH 5,3) gegeben und 21 Stunden bei Raumtemperatur geschüttelt. Danach wurde der Träger zweimal 5 Minuten und einmal 1 Stunde in je 50 ml 0,0025 M wäßriger Natriumacetatlösung (pH = 5,3) gewaschen.

Zur Ermittlung der Enzymaktivität ("immobiliserten Aktivität") wurde das Polymerisat in 50 ml einer Lösung von 17,5 g Sucrose in 0,01 M wäßriger Natriumacetatlösung (pH = 5,3) geschüttelt. Die Hydrolyse der Sucrose wurde polarimetrisch ermittelt.

Es ergab sich eine immobilisierte Aktivität von 45,3 g Sucrose pro Gramm Träger und Stunde.

Vergleichsbeispiel — Träger mit vernetztem Polyethylenimin

10 g Trägermaterial, hergestellt nach Beispiel 1 A) bis C), wurden in einem Rundkolben mit Rührer, Rückflußkühler und Trockenrohr (gefüllt mit Calciumchlorid) zusammen mit 100 ml Lösung von 10 g Epikote 828 (Shell) in wasserfreiem Aceton 5 Stunden bei 50°C gerührt. Dann wurde die Lösung auf einer Glasfritte vom Trägermaterial abgesaugt und der Träger bei 70°C im Vakuum getrocknet. Die Beladung des Trägers mit Invertase und die anschließende Prüfung der gebundenen Invertase-Aktivität entsprechend Beispiel 1 D) ergab eine Aktivität von 23,4 g Sucrose/g Träger.h. Man erkennt hier die im Vergleich zum erfindungsgemäßen Träger geringere Aktivität.

### Beispiel 2

0,5 g Träger entsprechend Beispiel 1 wurden in eine Säule mit einem Durchmesser von 1,6 cm gefüllt. Dann wurde die Lösung von 500 mg ß-Fructosidase in 100 ml 0,05 M Natriumacetatpuffer (pH 5,3) mit einer Förderrate von 100 ml/h 16 Stunden lang bei Raumtemperatur im Kreislauf durch die Säule gepumpt. Danach wurden zur Entfernung von nicht gebundenem Enzym 24 Stunden lang 100 ml wäßriger 0,05 M Natriumacetatlösung (pH 5,3) pro Stunde durch die Säule gepumpt. Anschließend wurde mit einer Förderrate von 86 ml/h bei 30°C eine Lösung von 350 g Sucrose/l in 0,05 M wäßriger Natriumacetatlösung (pH 5,3) durch die Säule gepumpt. Die Hydrolyse der Sucrose wurde polarimetrisch ermittelt. Zu Beginn wurden pro Gramm Träger, beladen mit ß-Fructosidase, und pro Stunde 27,5 g Sucrose hydrolysiert. Nach 70 Tagen besaß der Träger noch 57 Prozent seiner anfänglichen immobilisierten Aktivität.

### Beispiel 3

0,2 g des wie in Beispiel 2 eingesetzten Trägers wurden 64 Stunden bei 4°C in 20 ml Lösung von 100 mg ß-Amylase (28 Internat. Units/mg) in 0,05 M wäßriger Natriumacetatlösung (pH 4,8) geschüttelt. Dann wurde der Träger jeweils bei Raumtemperatur zweimal 5 Minuten und zweimal 1 Stunde in je 50 ml 0,05 M wäßriger Natriumacetatlösung von pH 4,8 gewaschen. Zur Ermittlung der immobilisierten enzymatischen Aktivität wurde der Träger bei 30°C 30 Minuten in 50 ml Lösung von 5 g Stärke nach Zulkowsky (Merck) in 0,016 M wäßriger Natriumacetatlösung (pH 4,8) geschüttelt. Die gebildete Maltose wurde mit 3,5-Dinitrosalicylsäure nach P. Berenfeld (Methods in Enzymology, Vol. 5, 149; Academic Press, 1955) bestimmt. Aktivität des Trägers: 1,1 g gebildete Maltose pro g und pro Stunde.

### Beispiel 4

Analog Beispiel 3 wurde Amyloglycosidase an den Träger gebunden. Aktivität des Trägers: 0,6 g gebildete Glucose pro g und pro Stunde.

### Patentansprüche

1. Makroporöser, hydrophiler kleinteiliger Träger für Enzyme aus einem mit Polyethylenimin beladenen Polymerisat, dadurch gekennzeichnet, daß das Polymerisat Sulfogruppen enthält und das Polyethylenimin weder vernetzt noch kovalent an das Polymerisat gebunden ist.

2. Träger nach Anspruch 1, dadurch gekennzeichnet, daß das sulfogruppenhaltige Polymerisat aus einem nach der Polymerisation sulfonierten Polymerisat aus folgenden Monomeren besteht:

a) 10 bis 100 Gew.% Divinylbenzol,

b) 0 bis 90 Gew.% Styrol, das ein- oder mehrfach $C_{1-4}$-alkylsubstituiert sein kann und

c) 0 bis 20 Gew.% mit Styrol und Divinylbenzol copolymerisierbarer Monomerer.

3. Träger nach Anspruch 1, dadurch gekennzeichnet, daß er ein Porenvolumen von 0,8 bis 4 cm³/g und Porendurchmesser von $2,5.10^{-5}$ bis $2.10^{-3}$ mm aufweist.

4. Träger nach Anspruch 2, dadurch gekennzeichnet, daß er ein Porenvolumen von 0,8 bis 4 cm³/g und Porendurchmesser von $2,5.10^{-5}$ bis $2.10^{-3}$ mm aufweist.

5. Träger nach Anspruch 1, dadurch gekennzeichnet, daß das verwendete Polyethylenimin ein Molekulargewicht von 1000 bis 50 000 besitzt.

6. Verwendung der Träger nach Anspruch 1 bis 4 zur Immobilierung von Enzymen.

### Revendications

1. Support hydrophile macroporeux en fines particules pour les enzymes, à base d'un polymère chargé de polyéthylène-imine, caractérisé en ce que le polymère comprend des groupes sulfo et al polyéthylène-imine n'est ni réticulée, ni liée par covalence au polymère.

2. Support suivant la revendication 1, caractérisé en ce que le polymère à groupes sulfo est composé des monomères ci-après:

a) 10 à 100% en poids de divinyl-benzène,

b) 0 à 90% en poids de styrène pouvant être substitué par un ou plusieurs radicaux alkyle en $C_1$ à $C_4$ et

c) 0 à 20% en poids de monomères copolymérisables avec le styrène et le divinyl-benzène, dont la polymérisation est suivie d'une sulfonation.

3. Support suivant la revendication 1, caractérisé en ce qu'il possède des pores d'un diamètre compris entre $2,5.10^{-3}$ et $2.10^{-3}$ mm, dont le volume est compris entre 0,8 et 4 $cm^3/g$.

4. Support suivant la revendication 2, caractérisé en ce qu'il possède des pores d'un diamètre compris entre $2,5.10^{-3}$ et $2.10^{-3}$ mm, dont le volume est compris entre 0,8 et 4 $cm^3/g$.

5. Support suivant la revendication 1, caractérisé en ce que la polyéthylène-imine utilisée possède un poids moléculaire de 1000 à 50 000.

6. Utilisation de supports suivant les revendications 1 à 4 pour l'immobilisation d'enzymes.

## Claims

1. A macroporous, hydrophilic, finely divided enzyme carrier of a polymer charged with polyethyleneimine, wherein the polymer contains sulfo groups and the polyethyleneimine is neither crosslinked nor covalently bonded to the polymer.

2. A carrier as claimed in claim 1, wherein the sulfo-containing polymer is a polymer, sulfonated after polymerization, of the following monomers:

a) from 10 to 100% by weight of divinylbenzene,

b) from 0 to 90% by weight of styrene, which may be mono- or polysubstituted by $C_1$—$C_4$-alkyl, and

c) from 0 to 20% by weight of monomers which are copolymerizable with styrene and divinylbenzene.

3. A carrier as claimed in claim 1, which has a pore volume of from 0.8 to 4 $cm^3/g$ and a pore diameter of from $2.5 \times 10^{-5}$ to $2 \times 10^{-3}$ mm.

4. A carrier as claimed in claim 2, which has a pore volume of from 0.8 to 4 $cm^3/g$ and a pore diameter of from $2.5 \times 10^{-3}$ mm.

5. A carrier as claimed in claim 1, wherein the polyethyleneimine used has a molecular weight of from 1,000 to 50,000.

6. Use of a carrier as claimed in any of claims 1 to 4 for immobilizing an enzyme.